# NEW EUROPEAN PATENT SPECIFICATION

(11) **EP 2 846 759 B2**
(45) Date of publication and mention of the opposition decision: **21.06.2023**
(45) Mention of the grant of the patent: 22.11.2017
(21) Application number: 13723054.6
(22) Date of filing: 06.05.2013
(51) Int. Cl.: A61Q 5/02, A61K 8/04, A61K 8/19, A61K 8/73, A61K 8/02, A61K 8/89, A61K 8/34

(54) **AEROSOL DEVICE BASED ON SEBUM-ABSORBING POWDER AND A PARTICULAR WATER-INSOLUBLE MINERAL COMPOUND**
AEROSOLVORRICHTUNG AUF BASIS EINES SEBUM-ABSORBIERENDEN PULVERS UND EINER BESTIMMTEN WASSERUNLÖSLICHEN MINERALVERBINDUNG
DISPOSITIF D'AÉROSOL À BASE DE POUDRE ABSORBANT LE SÉBUM ET D'UN COMPOSÉ MINÉRAL PARTICULIER INSOLUBLE DANS L'EAU

(30) Priority: 07.05.2012 FR 1254170; 29.06.2012 US 201261666784 P
(43) Date of publication of application: 18.03.2015
(73) Proprietor: L'OREAL, 75008 Paris (FR)
(72) Inventor: GAWTREY, Jonathan, 92100 Boulogne (FR); SMAIL, Nadia, 78540 Vernouillet (FR)
(74) Representative: Casalonga
(86) International application number: PCT/EP2013/059393
(87) International publication number: WO 2013/167536

(56) References cited:
- WO-A1-98/43599
- WO-A1-2011/056625
- WO-A2-03/049711
- WO-A2-2011/019539
- GB-A- 1 218 222
- US-A- 3 161 460
- US-A- 5 223 244
- DATABASE GNPD [Online] MINTEL; April 2010 (2010-04), "Refresh Dry Shampoo", XP002690820, Database accession no. 1317319
- DATABASE GNPD [Online] MINTEL; June 2011 (2011-06), "Brown Hair Powder Shampoo", XP002690821, Database accession no. 1555610

## Description

The present invention relates to an aerosol device comprising a cosmetic composition based on at least one sebum-absorbing powder and on at least one particular water-insoluble mineral compound, and to its use for the dry washing and cosmetic treatment of keratin materials, preferably human keratin fibres such as the hair.

In the field of washing keratin materials, dry shampoos have existed for many years, either in a powdery form or in aerosol form. They enable excess sebum to be removed quickly without wetting the head of hair. They act by taking up sebum by absorption by means of powders chosen for their sebum-absorbing qualities.

The powders used may be of mineral, organic or synthetic origin and may be wheat, rice and corn starch derivatives.

Aerosol dry shampoos are described for example in WO 2011/056625. They comprise a carrier material, a starch material such as aluminium starch octenylsuccinamate, a clay material such as steralkonium hectorite, and a propellant.

In practice, the proposed dry shampoos are not entirely satisfactory. Their styling properties, such as the provision of volume to the head of hair and the lifting of the roots, are generally very limited and they very often leave white residues on the hair.

There is thus a need to develop novel dry shampoo compositions which offer both optimum cleansing activity and provision of volume to the head of hair.

The Applicant has found, surprisingly and advantageously, that the use of at least one sebum-absorbing powder in combination with at least one particular water-insoluble mineral compound, optionally in combination with a particular silicone gum, makes it possible to offer the cleansing properties expected of dry shampoos and also styling properties such as a provision of volume and body to the head of hair.

Moreover, the compositions of the invention leave less white residue than the products conventionally used.

One subject of the invention is thus an aerosol device which contains a cosmetic composition comprising:
(i) one or more sebum-absorbing powders with a sebum uptake of greater than or equal to 35 ml/100 g,
(ii) a water-insoluble mineral compound which is calcium carbonate,
(iii) one or more C₂₋₄ monoalcohols, and
(iv) one or more propellants.

These particular combinations make it possible to obtain a cleansing effect and also a styling effect, especially for providing volume and lifting of the roots.

Moreover, they leave less white residue while at the same time being more styling than the known products.

The present invention also relates to a process for the dry washing and cosmetic treatment of keratin materials, especially human keratin fibres such as the hair, comprising the spraying onto the keratin materials of the compositions according to the invention.

The invention also relates to the use of the cosmetic compositions sprayed from this aerosol device for the dry washing and cosmetic treatment of keratin materials, preferably human keratin fibres and better still the hair.

Other subjects, characteristics, aspects and advantages of the invention will become even more clearly apparent on reading the description and the example that follows.

In the text hereinbelow, and unless otherwise indicated, the limits of a range of values are included within this range, in particular in the expressions "between" and "ranging from ... to ...".

Moreover, the expression "at least one" used in the present description is equivalent to the expression "one or more".

According to the invention, the aerosol device contains a cosmetic composition comprising:
(i) one or more sebum-absorbing powders with a sebum uptake of greater than or equal to 35 ml/100 g,
(ii) a water-insoluble mineral compound which is calcium carbonate,
(iii) one or more C₂₋₄ monoalcohols, and
(iv) one or more propellants.

For the purposes of the present invention, the term "sebum-absorbing powder" means a powder that is capable of absorbing and/or adsorbing sebum, which has a sebum uptake of greater than or equal to 35 ml/100 g.

The sebum uptake corresponds to the amount of sebum absorbed and/or adsorbed by the powder. It is expressed in ml of sebum per 100 g of powder and measured by means of the powder oil uptake determination method described in standard NF T 30-022.

The powder oil uptake corresponds to the amount of sebum absorbed onto the available surface of the powder by measuring the "wet point" as indicated below.

The measuring method is as follows: an amount m (in grams) of between 0.5 and 5 g of powder is placed on a glass plate, the amount depending on the density of the powder, followed by dropwise addition of artificial sebum having the following composition:

| | |
|---|---|
| - triolein | 29% by weight |
| - oleic acid | 28.5% by weight |
| - oleyl oleate | 18.5% by weight |
| - squalene | 14% by weight |
| - cholesterol | 7% by weight |
| - cholesteryl palmitate | 3% by weight |

After addition of 4 to 5 drops of artificial sebum, the artificial sebum is incorporated into the powder using a spatula, and the addition of the artificial sebum is continued until conglomerates of artificial sebum and of powder form. At this point, the artificial sebum is added one drop at a time and the mixture is then triturated with the spatula.

The addition of artificial sebum is stopped when a firm, smooth paste is obtained. This paste must be able to be spread over the glass plate without cracks or the formation of lumps. The volume Vs in ml of artificial sebum used is then noted.

The sebum uptake corresponds to the ratio Vs/m.

The sebum-absorbing powder(s) used in the aerosol device of the invention have a sebum uptake preferably ranging from 35 to 1000 ml/100 g and better still from 35 to 800 ml/100 g.

Advantageously, the sebum-absorbing particle may have a BET specific surface area of greater than or equal to 150 m²/g, preferably greater than or equal to 300 m²/g, better still greater than 500 m²/g and preferentially greater than 600 m²/g, and especially less than 1500 m²/g.

The BET specific surface area is determined according to the BET (Brunauer-Emmet-Teller) method described in the Journal of the American Chemical Society, vol. 60, page 309, February 1938 and corresponding to the international standard ISO 5794/1 (appendix D). The BET specific surface area corresponds to the total specific surface area (thus including micropores) of the particle and especially of the powder.

The sebum-absorbing powder may be a mineral powder or an organic powder.

More precisely, the sebum-absorbing powder may be chosen from:
starches,
calcium silicates,
perlites,
zeolites,
polylactic acids,
silicas,
polyamide (Nylon^{®}) powders,
powders of acrylic polymers, especially of polymethyl methacrylate, poly(methyl methacrylate/ethylene glycol dimethacrylate), poly(allyl methacrylate/ethylene glycol dimethacrylate), ethylene glycol dimethacrylate/lauryl methacrylate copolymer;
powders of silicone elastomer, obtained especially by polymerization of organopolysiloxane containing at least two hydrogen atoms each bonded to a silicon atom and of an organopolysiloxane comprising at least two ethylenically unsaturated groups (especially two vinyl groups) in the presence of a platinum catalyst; and
mixtures thereof.

The sebum-absorbing powder may be a powder coated with a hydrophobic treatment agent.

The hydrophobic treatment agent may be chosen from fatty acids, for instance stearic acid; metal soaps, for instance aluminium dimyristate, the aluminium salt of hydrogenated tallow glutamate; amino acids; N-acylamino acids or salts thereof; lecithin, isopropyl triisostearyl titanate, and mixtures thereof.

The N-acylamino acids may comprise an acyl group containing from 8 to 22 carbon atoms, for instance a 2-ethylhexanoyl, caproyl, lauroyl, myristoyl, palmitoyl, stearoyl or cocoyl group. The salts of these compounds may be the aluminium, magnesium, calcium, zirconium, zinc, sodium or potassium salts. The amino acid may be, for example, lysine, glutamic acid or alanine.

The term "alkyl" mentioned in the compounds cited above especially denotes an alkyl group containing from 1 to 30 carbon atoms and preferably containing from 5 to 16 carbon atoms.

The starches that may be used in the present invention are, for example, corn starch, potato starch, tapioca starch, rice starch, wheat starch and cassava starch.

The starches may be modified or unmodified.

A modified starch is a starch that has been modified via processes known to those skilled in the art, for instance esterification, etherification, oxidation, acid hydrolysis, crosslinking or enzymatic conversion.

Non-limiting examples of modified starches include aluminium starch octenylsuccinate, sodium starch octenylsuccinate, calcium starch octenylsuccinate, distarch phosphate, hydroxyethyl starch phosphate, hydroxypropyl starch phosphate, sodium carboxymethyl starch and sodium starch glycolate.

In one particular embodiment, the starch is a starch octenylsuccinate, in particular of aluminium, the starch being from corn, wheat or rice. Mention may be made especially of the product sold by Akzo Nobel under the name Dry Flo Plus.

Preferably, the calcium silicates used as sebum-absorbing powder have a sebum uptake of greater than 200 ml/100 g, better still between 400 ml/100 g and 600 ml/100 g and more preferentially of about 475 ml/100 g.

The specific surface area (BET) preferably ranges from about 150 m²/g to 600 m²/g, better still from 300 m²/g to 600 m²/g and even more preferentially from 310 m²/g to 350 m²/g.

The size of the silicate particles is preferably less than 20 micrometres.

These calcium silicates are generally prepared by reaction of reactive silica with an alkaline-earth metal reagent, preferably an alkaline-earth metal oxide or hydroxide, and a source of aluminium such as sodium aluminate or alumina. Since the final properties of the silicate are functions of the reactivity of the silica, the preferred source of silica is the product of reaction of a soluble silicate, such as sodium silicate, and of a mineral acid such as sulfuric acid. Suitable amorphous synthetic alkaline-earth metal silicates are manufactured by the company JM Huber Corporation and are sold under the name Hubersorb^{®}. Methods for preparing these silicas are disclosed in greater detail in patent US 4 557 916. Other suitable silicates are available from JM Huber Corporation, for instance the sodium aluminosilicate sold under the brand name Zeolexg and the sodium magnesium aluminosilicate sold under the brand name Hydrex^{®}.

Sebum-absorbing powders that may also be used include perlites, which are generally aluminosilicates of volcanic origin and whose composition is as follows:
70.0-75.0% by weight of silica SiO₂
12.0-15.0% by weight of aluminium oxide Al₂O₃
3.0-5.0% of sodium oxide Na₂O
3.0-5.0% of potassium oxide K₂O
0.5-2% of iron oxide Fe₂O₃
0.2-0.7% of magnesium oxide MgO
0.5-1.5% of calcium oxide CaO
0.05-0.15% of titanium oxide TiO₂.

Examples of zeolites that may especially be mentioned include sodium or potassium aluminosilicate compounds such as the product sold by Zeochem under the name Xmol.

The polylactic acids that may be used in the present invention are especially Accurel EP600 from Akzo Nobel or the product sold under the name Lactic Acid Polymer 9105 by Dajac Labs.

Silica powders that may be mentioned include:
- the porous silica microspheres sold under the name Silica Beads SB-700 by the company Miyoshi; Sunsphere^{®} H51, Sunsphere^{®} H33 by the company Asahi Glass;
- the polydimethylsiloxane-coated amorphous silica microspheres sold under the name SA Sunsphere^{®} H33 and SA Sunsphere^{®} H53 by the company Asahi Glass.

A Nylon powder that may be mentioned is the Nylon powder sold under the name Orgasol^{®} 4000 by the company Atochem.

Acrylic polymer powders that may be mentioned include:
- the polymethyl methacrylate powders sold under the name Covabead^{®} LH85 by the company Wackherr;
- the polymethyl methacrylate/ethylene glycol dimethacrylate powders sold under the name Dow Corning 5640 Microsponge^{®} Skin Oil Adsorber by the company Dow Corning; Ganzpearl^{®} GMP-0820 by the company Ganz Chemical;
- the polyallyl methacrylate/ethylene glycol dimethacrylate powders sold under the name Poly-Pore^{®} L200 or Poly-Pore^{®} E200 by the company Amcol Health and Beauty Solutions Inc.; these powders especially have a sebum uptake of greater than or equal to 1 ml/g, better still ranging from 1 ml/g to 20 ml/g;
- the ethylene glycol dimethacrylate/lauryl methacrylate copolymer powders sold under the name Polytrap^{®} 6603 from the company Dow Corning.

Silicone elastomer powders that may be mentioned include the powders sold under the names Trefil^{®} Powder E-505C and Trefil^{®} Powder E-506C by the company Dow Corning.

Preferably, the sebum-absorbing powder is chosen from modified starches such as starch octenylsuccinates, in particular of aluminium, perlite, polylactic acids and zeolites, and better still from starch octenylsuccinates.

The sebum-absorbing powder(s) are present in an amount preferably ranging from 0.1% to 30% by weight, better still from 1% to 15% by weight and even more preferentially from 2% to 12% by weight relative to the total weight of the composition.

The water-insoluble mineral compound used in the compositions of the invention is calcium carbonate.

For the purposes of the present invention, the term "water-insoluble" refers to a compound whose solubility at spontaneous pH in water at 25°C and at atmospheric pressure is less than 0.1%.

Preferably, this compounds has a mean particle size of from 20 to 50 µm, as water-insoluble mineral compound.

The water-insoluble mineral compound is present in an amount preferably ranging from 0.1% to 30% by weight, better still from 0.5% to 15% by weight and even more preferentially from 1% to 10% by weight relative to the total weight of the composition.

As C₂₋₄ monoalcohol(s) that may be used in the aerosol device of the invention, mention may be made especially of ethanol or isopropanol, and better still ethanol.

The C₂₋₄ monoalcohol(s) are preferably present in an amount ranging from 2% to 70% by weight, better still from 5% to 60% by weight and even more preferentially from 10% to 50% by weight relative to the total weight of the composition.

The compositions according to the invention may contain one or more additional organic solvents such as polyols, for instance glycerol, propylene glycol or polyethylene glycols.

They may also contain water.

Preferably, the compositions according to the invention contain less than 5% by weight of water relative to the total weight of the composition. Even more preferentially, they do not contain any added water. The compositions are then said to be anhydrous.

Examples of propellants that may be used in the aerosol device of the present invention are liquefied gases such as dimethyl ether, 1,1-difluoroethane, or C₃₋₅ alkanes, for instance propane, isopropane, n-butane, isobutane or pentane, or compressed gases such as air, nitrogen or carbon dioxide, and mixtures thereof.

Mention may be made preferentially of C₃₋₅ alkanes and in particular propane, n-butane and isobutane, and mixtures thereof.

The propellant(s) are preferably present in an amount ranging from 10% to 90% by weight, better still from 15% to 80% by weight and even more preferentially from 20% to 75% by weight relative to the total weight of the composition.

Preferably, the compositions of the invention may also comprise one or more high-viscosity silicone gums.

The high-viscosity silicone gums used in the invention generally have a viscosity of greater than or equal to 0.5 × 10⁻³ m²/s (500 cSt), preferably ranging from 1 × 10⁻³ to 10 × 10⁻³ m²/s. The viscosity is measured using a Brookfield viscometer at 25°C.

The silicone gums that may be present in the composition according to the invention are especially polydiorganosiloxanes, used alone or as a mixture in a solvent. This solvent can be chosen from volatile silicones, polydimethylsiloxane (PDMS) oils, polyphenylmethylsiloxane (PPMS) oils, isoparaffins, polyisobutylenes, methylene chloride, pentane, dodecane and tridecane, or mixtures thereof.

Examples of silicone gums that may be mentioned more particularly include the following products:
- polydimethylsiloxane gums,
- α,ω-disilanol polydimethylsiloxane gums,
- polydimethylsiloxane/methylvinylsiloxane gums,
- polydimethylsiloxane/diphenylsiloxane gums,
- polydimethylsiloxane/phenylmethylsiloxane gums,
- polydimethylsiloxane/diphenylsiloxane/methylvinylsiloxane gums.

Products that may be used more particularly are the following mixtures:
- mixtures formed from a polydimethylsiloxane hydroxylated at the end of the chain (known as dimethiconol according to the nomenclature of the CTFA dictionary) and from a linear polydimethylsiloxane (known as dimethicone according to the nomenclature of the CTFA dictionary), such as the product Mirasil D-DML sold by the company Dow Corning,
- mixtures formed from a polydimethylsiloxane hydroxylated at the end of the chain (known as dimethiconol according to the nomenclature of the CTFA dictionary) and from a cyclic polydimethylsiloxane (known as cyclomethicone according to the nomenclature of the CTFA dictionary), such as the product Q2 1401 sold by the company Dow Corning.

When the high-viscosity silicone gum(s) are present in the device of the invention, their amount preferably ranges from 0.05% to 5% by weight relative to the total weight of the composition.

The compositions of the invention also optionally comprise one or more fatty esters.

The fatty esters optionally used in the invention are liquid or non-liquid.

The term "liquid fatty ester" means an ester derived from a fatty acid and/or from a fatty alcohol that is liquid at standard temperature (25°C) and at atmospheric pressure (760 mmHg, i.e. 1.013 × 10⁵ Pa).

The esters are preferably liquid esters of saturated or unsaturated, linear or branched C₁-C₂₆ aliphatic monoacids or polyacids and of saturated or unsaturated, linear or branched C₁-C₂₆ aliphatic monoalcohols or polyalcohols, the total number of carbon atoms of the esters being greater than or equal to 10.

Preferably, for the esters of monoalcohols, at least one of the alcohol or of the acid from which the esters of the invention result is branched.

Among the monoesters of monoacids and of monoalcohols, mention may be made of ethyl palmitate, isopropyl palmitate, alkyl myristates, preferably of C₂-C₂₂, such as isopropyl myristate or ethyl myristate, isocetyl stearate, 2-ethylhexyl isononanoate, isononyl isononanoate, isodecyl neopentanoate and isostearyl neopentanoate.

Esters of C₄-C₂₂ dicarboxylic or tricarboxylic acids and of C₁-C₂₂ alcohols and esters of monocarboxylic, dicarboxylic or tricarboxylic acids and of C₄-C₂₆ dihydroxy, trihydroxy, tetrahydroxy or pentahydroxy non-sugar alcohols may also be used.

Mention may be made especially of: diethyl sebacate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; diethylene glycol diisononanoate.

The compositions may also comprise, as liquid fatty ester, sugar esters and diesters of C₆-C₃₀ and preferably C₁₂-C₂₂ fatty acids. It is recalled that the term "sugar" means oxygen-bearing hydrocarbon-based compounds containing several alcohol functions, with or without aldehyde or ketone functions, and which comprise at least 4 carbon atoms. These sugars can be monosaccharides, oligosaccharides or polysaccharides.

Examples of suitable sugars that may be mentioned include sucrose, glucose, galactose, ribose, fucose, maltose, fructose, mannose, arabinose, xylose and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may have one to three conjugated or unconjugated carbon-carbon double bonds.

The esters according to this variant may also be chosen from mono-, di-, tri- and tetraesters, and polyesters, and mixtures thereof.

These esters may be, for example, oleates, laurates, palmitates, myristates, behenates, cocoates, stearates, linoleates, linolenates, caprates and arachidonates, or mixtures thereof, such as, in particular, oleopalmitate, oleostearate or palmitostearate mixed esters.

More particularly, use is made of monoesters and diesters and in particular of sucrose, glucose or methylglucose mono- or dioleates, stearates, behenates, oleopalmitates, linoleates, linolenates or oleostearates.

An example that may be mentioned is the product sold under the name Glucate^{®} DO by the company Amerchol, which is a methylglucose dioleate.

Finally, use may also be made of natural or synthetic glycerol esters of mono-, di- or triacids.

Among these, mention may be made of plant oils.

As oils of plant origin or synthetic triglycerides that may be used in the composition of the invention as liquid fatty esters, examples that may be mentioned include:
- triglyceride oils of plant or synthetic origin, such as liquid fatty acid triglycerides containing from 6 to 30 carbon atoms, for instance heptanoic or octanoic acid triglycerides, or alternatively, for example, sunflower oil, corn oil, soybean oil, marrow oil, grapeseed oil, sesame seed oil, hazelnut oil, apricot oil, macadamia oil, arara oil, castor oil, avocado oil, olive oil, rapeseed oil, coconut oil, wheatgerm oil, sweet almond oil, safflower oil, candlenut oil, camellina oil, tamanu oil, babassu oil and pracaxi oil, caprylic/capric acid triglycerides, for instance those sold by the company Stéarineries Dubois or those sold under the names Miglyol^{®} 810, 812 and 818 by the company Dynamit Nobel, jojoba oil and shea butter oil.

Fatty esters that will preferably be used include liquid fatty esters derived from monoalcohols, such as isopropyl myristate or palmitate, and more particularly isopropyl myristate.

The non-liquid fatty esters are especially solid esters derived from C₉-C₂₆ fatty acids and from C₉-C₂₆ fatty alcohols.

Among these esters, mention may be made of octyldodecyl behenate, isocetyl behenate, cetyl lactate, stearyl octanoate, octyl octanoate, cetyl octanoate, decyl oleate, myristyl stearate, octyl palmitate, octyl pelargonate, octyl stearate, alkyl myristates such as cetyl myristate, myristyl myristate and stearyl myristate, and hexyl stearate.

Esters of C₄-C₂₂ dicarboxylic or tricarboxylic acids and of C₁-C₂₂ alcohols and esters of monocarboxylic, dicarboxylic or tricarboxylic acids and of C₂-C₂₆ dihydroxy, trihydroxy, tetrahydroxy or pentahydroxy alcohols may also be used.

Examples that may especially be mentioned include diethyl sebacate, diisopropyl sebacate, diisopropyl adipate, di-n-propyl adipate, dioctyl adipate and dioctyl maleate.

Among all the esters mentioned above, it is preferred to use myristyl, cetyl or stearyl palmitates, and alkyl myristates such as cetyl myristate, stearyl myristate and myristyl myristate.

When the fatty ester(s) are present in the device of the invention, their amount preferably ranges from 0.1% to 30% by weight and better still from 1% to 10% by weight relative to the total weight of the composition.

The compositions contained in the aerosol device of the invention may also comprise propylene carbonate, preferably in an amount ranging from 0.05% to 5% by weight relative to the total weight of the composition.

The compositions defined in the invention may also contain one or more additives chosen from conditioning or fixing anionic, cationic, nonionic, amphoteric or zwitterionic polymers, fragrances, dyes, protective screening agents, acids, bases, nacres and glitter flakes.

These additives may be present in the composition according to the invention in an amount ranging from 0 to 20% by weight, relative to the total weight of the composition.

A person skilled in the art will take care to select these optional additives and amounts thereof so that they do not harm the properties of the compositions of the present invention.

The compositions in accordance with the invention are conditioned in an aerosol device that is common in cosmetics.

The aerosol device serving for conditioning the composition of the invention may consist of an outer aerosol can which contains both the propellant(s) and the other ingredients of the composition in a single compartment. Preferably, the said device is equipped with a valve with an inner restriction of 0.3 to 3 mm and better still from 0.5 to 1.5 mm, an inner nozzle of 0.3 to 3 mm and better still from 0.5 to 1.5 mm, and an additional gas intake of 0.3 to 3 mm and better still from 0.5 to 1.5 mm. The device also comprises a push button which is equipped with a direct outlet orifice with a diameter of between 0.25 and 1 mm and preferably between 0.4 and 0.7 mm.

According to another variant, the aerosol device may be in two compartments, formed from an outer aerosol can comprising an inner bag hermetically welded to a valve. The various ingredients of the composition are introduced into the inner bag and a compressed gas is introduced between the bag and the can at a sufficient pressure to make the product come out in the form of a spray through a nozzle orifice. Preferably, the said device is equipped with a valve with an inner restriction of 0.3 to 3 mm and better still from 0.5 to 1.5 mm, an inner nozzle of 0.3 to 3 mm and better still from 0.5 to 1.5 mm. The device also comprises a push button which is equipped with a direct outlet orifice with a diameter of between 0.25 and 1 mm and preferably between 0.4 and 0.7 mm. Such a device is sold, for example, under the name EP Spray by the company EP-Spray System SA. The said compressed gas is preferably used at a pressure of between 1 and 12 bar and better still between 9 and 11 bar.

The sprayed compositions are in the form of a spray.

The present invention also relates to a process for the dry washing and cosmetic treatment of the hair, comprising the use of a cosmetic composition as described above.

In particular, the process for the dry washing and cosmetic treatment of the hair comprises a step of applying to dry hair a cosmetic composition as defined previously, sprayed from an aerosol device according to the invention.

The present invention also relates to the use of the cosmetic compositions defined above sprayed from the aerosol device according to the invention, for the dry washing and cosmetic treatment of the hair.

The example that follows serves to illustrate the invention.

### EXAMPLE

In the example that follows, all the amounts are indicated as weight percentages of product as active materials relative to the total weight of the composition.

The composition below according to the invention was prepared from the compounds indicated in the table below.

| | Amount in weight% |
|---|---|
| Aluminium Starch Octenylsuccinate¹ | 10% |
| Calcium carbonate (D50 = 35 µm)² | 2% |
| Hectorite modified with distearyldimethylammonium chloride³ | 0.25% |
| Dimethicone/dimethiconol⁴ | 0.55% |
| Isopropyl myristate | 2% |
| Propylene carbonate⁵ | 0.07% |
| Fragrance | 0.2% |
| Ethanol | 14.93% |
| Isobutane | 70% |
| ¹: Sold under the trade name Dry Flo Plus by National Starch | |
| ²: Sold under the trade name Omyacare S60 by Omya | |
| ³: Sold under the trade name Bentone 38 by Elementis | |
| ⁴: Sold under the trade name Mirasil D-DML by Dow Corning | |
| ⁵: Sold under the trade name Jeffsol Propylene Carbonate by Huntsman | |

This composition was then introduced into an aerosol device. The said device is equipped with a valve with an inner restriction of 0.8 mm, an inner nozzle of 0.6 mm, an additional gas intake of 0.4 mm and a push button with a direct outlet orifice of 0.49 mm.

The lacquer was sprayed onto a head of greasy, dirty hair.

After washing, it is found that the head of hair is visibly cleaner, with little visible residue. Lifting of the roots and provision of volume are also observed.

## Claims

1. Aerosol device which contains a cosmetic composition comprising:
(i) one or more sebum-absorbing powders with a sebum uptake of greater than or equal to 35 ml/100 g,
(ii) a water-insoluble mineral compound which is calcium carbonate,
(iii) one or more C₂₋₄ monoalcohols, and
(iv) one or more propellants.

2. Aerosol device according to Claim 1, **characterized in that** the sebum-absorbing powder is chosen from modified starches such as starch octenylsuccinates, in particular of aluminium, perlite, polylactic acids and zeolites, and better still from starch octenylsuccinates.

3. Aerosol device according to Claim 1 or 2, **characterized in that** the sebum-absorbing powder(s) are present in an amount ranging from 0.1% to 30% by weight, preferably from 1% to 15% by weight and even more preferentially from 2% to 12% by weight relative to the total weight of the composition.

4. Aerosol device according to any one of the preceding claims, **characterized in that** the water-insoluble mineral compound is present in an amount ranging from 0.1% to 30% by weight, better still from 0.5% to 15% by weight and even more preferentially from 1% to 10% by weight relative to the total weight of the composition.

5. Aerosol device according to any one of the preceding claims, **characterized in that** the C₂₋₄ monoalcohol is ethanol.

6. Aerosol device according to any one of the preceding claims, **characterized in that** the C₂₋₄ monoalcohol(s) are present in an amount ranging from 2% to 70% by weight, better still from 5% to 60% by weight and even more preferentially from 10% to 50% by weight relative to the total weight of the composition.

7. Aerosol device according to any one of the preceding claims, **characterized in that** the propellant(s) are chosen from C₃₋₅ alkanes.

8. Aerosol device according to any one of the preceding claims, **characterized in that** the propellant(s) are present in an amount ranging from 10% to 90% by weight, better still from 15% to 80% by weight and even more preferentially from 20% to 75% by weight relative to the total weight of the composition.

9. Aerosol device according to any one of the preceding claims, **characterized in that** the composition comprises at least one silicone gum with a viscosity of greater than 0.5 × 10⁻³ m²/s (500 cSt).

10. Aerosol device according to any one of the preceding claims, **characterized in that** the composition comprises at least one fatty ester such as isopropyl myristate.

11. Aerosol device according to any one of the preceding claims, **characterized in that** the composition comprises at least one additive chosen from conditioning or fixing anionic, cationic, nonionic, amphoteric or zwitterionic polymers, fragrances, dyes, protective screening agents, acids, bases, nacres and glitter flakes.

12. Process for the dry washing and cosmetic treatment of keratin materials, comprising a step of applying to dry hair a cosmetic composition as defined in any one of Claims 1 to 11, sprayed from an aerosol device according to any one of Claims 1 to 11.

13. Use of the cosmetic composition sprayed from the aerosol device according to any one of Claims 1 to 11, for the dry washing and treatment of keratin materials.

## Patentansprüche

1. Aerosolvorrichtung, die eine kosmetische Zusammensetzung enthält, welche umfasst:
(i) ein oder mehrere talgabsorbierende Pulver mit einer Talgaufnahme von höher als oder gleich 35 ml/100 g,
(ii) eine wasserunlösliche Mineralverbindung, die Calciumcarbonat ist,
(iii) einen oder mehrere C₂₋₄Monoalkohole und
(iv) ein oder mehrere Treibmittel.

2. Aerosolvorrichtung gemäß Anspruch 1, **dadurch gekennzeichnet, dass** das talgabsorbierende Pulver ausgewählt ist aus modifizierten Stärken, wie z. B. Stärkeoctenylsuccinaten, insbesondere von Aluminium, Perlit, Polymilchsäuren und Zeolithen, noch besser aus Stärkeoctenylsuccinaten.

3. Aerosolvorrichtung gemäß Anspruch 1 oder 2, **dadurch gekennzeichnet, dass** das/die talgabsorbierende(n) Pulver in einer Menge in dem Bereich von 0,1 Gew.-% bis 30 Gew.-%, vorzugsweise von 1 Gew.-% bis 15 Gew.-% und noch bevorzugter von 2 Gew.-% bis 12 Gew.-%, bezogen auf das Gesamtgewicht der Zusammensetzung, vorhanden ist/sind.

4. Aerosolvorrichtung gemäß einem der vorstehenden Ansprüche, **dadurch gekennzeichnet, dass** die wasserunlösliche Mineralverbindung in einer Menge in dem Bereich von 0,1 Gew.-% bis 30 Gew.-%, noch besser von 0,5 Gew.-% bis 15 Gew.-% und noch bevorzugter von 1 Gew.-% bis 10 Gew.-%, bezogen auf das Gesamtgewicht der Zusammensetzung, vorhanden ist.

5. Aerosolvorrichtung gemäß einem der vorstehenden Ansprüche, **dadurch gekennzeichnet, dass** der C₂₋₄Monoalkohol Ethanol ist.

6. Aerosolvorrichtung gemäß einem der vorstehenden Ansprüche, **dadurch gekennzeichnet, dass** der/die C₂₋₄Monoalkohol(e) in einer Menge in dem Bereich von 2 Gew.-% bis 70 Gew.-%, noch besser von 5 Gew.-% bis 60 Gew.-% und noch bevorzugter von 10 Gew.-% bis 50 Gew.-%, bezogen auf das Gesamtgewicht der Zusammensetzung, vorhanden ist/sind.

7. Aerosolvorrichtung gemäß einem der vorstehenden Ansprüche, **dadurch gekennzeichnet, dass** das/die Treibmittel ausgewählt ist/sind aus Cs-sAlkanen.

8. Aerosolvorrichtung gemäß einem der vorstehenden Ansprüche, **dadurch gekennzeichnet, dass** das/die Treibmittel in einer Menge in dem Bereich von 10 Gew.-% bis 90 Gew.-%, noch besser von 15 Gew.-% bis 80 Gew.-% und noch bevorzugter von 20 Gew.-% bis 75 Gew.-%, bezogen auf das Gesamtgewicht der Zusammensetzung, vorhanden ist/sind.

9. Aerosolvorrichtung gemäß einem der vorstehenden Ansprüche, **dadurch gekennzeichnet, dass** die Zusammensetzung wenigstens einen Silikonkautschuk mit einer Viskosität von höher als 0,5 × 10⁻³ m²/s (500 cSt) umfasst.

10. Aerosolvorrichtung gemäß einem der vorstehenden Ansprüche, **dadurch gekennzeichnet, dass** die Zusammensetzung wenigstens einen Fettester, wie z. B. Isopropylmyristat, umfasst.

11. Aerosolvorrichtung gemäß einem der vorstehenden Ansprüche, **dadurch gekennzeichnet, dass** die Zusammensetzung wenigstens einen Zusatzstoff ausgewählt aus konditionierenden oder fixierenden anionischen, kationischen, nichtionischen, amphoteren oder zwitterionischen Polymeren, Duftstoffen, Farbstoffen, Sonnenschutzmitteln, Säuren, Basen, Perlglanzstoffen und Glitzerflocken, umfasst.

12. Verfahren zum Trockenwaschen und zur kosmetischen Behandlung von Keratinmaterialien, umfassend einen Schritt des Aufbringens einer kosmetischen Zusammensetzung gemäß einem der Ansprüche 1 bis 11 aus einer Aerosolvorrichtung gemäß einem der Ansprüche 1 bis 11 gesprüht auf trockenes Haar.

13. Verwendung der aus der Aerosolvorrichtung gemäß einem der Ansprüche 1 bis 11 gesprühten kosmetischen Zusammensetzung zum Trockenwaschen und zur Behandlung von Keratinmaterialien.

## Revendications

1. Dispositif aérosol qui contient une composition cosmétique comprenant :
(i) une ou plusieurs poudres absorbantes de sébum présentant une prise de sébum supérieure ou égale à 35 ml/100 g,
(ii) un composé minéral insoluble dans l'eau qui est le carbonate de calcium,
(iii) un ou plusieurs monoalcools en C₂₋₄, et
(iv) un ou plusieurs agents propulseurs.

2. Dispositif aérosol selon la revendication 1, **caractérisé en ce que** la poudre absorbante de sébum est choisie parmi les amidons modifiés tels que les octénylsuccinates d'amidon et en particulier d'aluminium, la perlite, les acides polylactiques, et les zéolithes, mieux parmi les octénylsuccinates d'amidons.

3. Dispositif aérosol selon la revendication 1 ou 2, **caractérisé en ce que** la ou les poudre(s) absorbante(s) de sébum est ou sont présente(s) en une quantité allant de 0,1 à 30 % en poids, de préférence de 1 à 15 % en poids, et encore plus préférentiellement de 2 à 12 % en poids par rapport au poids total de la composition.

4. Dispositif aérosol selon l'une quelconque des revendications précédentes, **caractérisé en ce que** le composé minéral insoluble dans l'eau est présent en une quantité allant de 0,1 à 30 % en poids, mieux encore de 0,5 à 15 % en poids, et encore plus préférentiellement de 1 à 10 % en poids par rapport au poids total de la composition.

5. Dispositif aérosol selon l'une quelconque des revendications précédentes, **caractérisé en ce que** le monoalcool en C₂₋₄ est l'éthanol.

6. Dispositif aérosol selon l'une quelconque des revendications précédentes, **caractérisé en ce que** le ou les monoalcools en C₂₋₄ est ou sont présent(s) en une quantité allant de 2 à 70 % en poids, mieux encore de 5 à 60% en poids, et encore plus préférentiellement de 10 à 50 % en poids par rapport au poids total de la composition.

7. Dispositif aérosol selon l'une quelconque des revendications précédentes, **caractérisé en ce que** l'agent ou les agents propulseur(s) est ou sont choisi(s) parmi les alcanes en C₃₋₅.

8. Dispositif aérosol selon l'une quelconque des revendications précédentes, **caractérisé en ce que** l'agent ou les agents propulseur(s) est ou sont présent(s) en une quantité allant de 10 à 90 % en poids, mieux encore de 15 à 80 % en poids, et encore plus préférentiellement de 20 à 75 % par rapport au poids total de la composition.

9. Dispositif aérosol selon l'une quelconque des revendications précédentes, **caractérisé en ce que** la composition comprend au moins une gomme de silicone de viscosité supérieure à 0,5 × 10⁻³ m²/s (500 cSt).

10. Dispositif aérosol selon l'une quelconque des revendications précédentes, **caractérisé en ce que** la composition comprend au moins un ester gras tel que le myristate d'isopropyle.

11. Dispositif aérosol selon l'une quelconque des revendications précédentes, **caractérisé en ce que** la composition comprend au moins un additif choisi parmi les polymères anioniques, cationiques, non ioniques, amphotères ou zwittérioniques conditionneurs ou fixants, les parfums, les colorants, les filtres protecteurs, les acides, les bases, les nacres, et les paillettes.

12. Procédé de lavage à sec et de traitement cosmétique des matières kératiniques, comportant une étape d'application sur les cheveux secs, d'une composition cosmétique telle que définie dans l'une quelconque des revendications 1 à 11, pulvérisée à partir d'un dispositif aérosol selon l'une quelconque des revendications 1 à 11.

13. Utilisation de la composition cosmétique pulvérisée à partir du dispositif aérosol selon l'une quelconque des revendications 1 à 11, pour le lavage à sec et le traitement des matières kératiniques.
